Europäisches Patentamt

European Patent Office

Office européen des brevets

(11) Veröffentlichungsnummer: **0 078 888**

**A1**

## (12) EUROPÄISCHE PATENTANMELDUNG

(21) Anmeldenummer: **82106704.8**

(22) Anmeldetag: **24.07.82**

(51) Int. Cl.³: **A 61 F 1/03**

(30) Priorität: **10.11.81 CH 7197/81**

(43) Veröffentlichungstag der Anmeldung:
**18.05.83 Patentblatt 83/20**

(84) Benannte Vertragsstaaten:
**AT DE FR GB IT NL**

(71) Anmelder: GEBRÜDER SULZER
AKTIENGESELLSCHAFT
Zürcherstrasse 9
CH-8401 Winterthur(CH)

(71) Anmelder: Protek AG
Stadtbachstrasse 64
CH-3000 Bern(CH)

(72) Erfinder: Müller, Maurice E., Prof. Dr.-med.
Inselspital
CH-3010 Bern(CH)

(74) Vertreter: Dipl.-Ing. H. Marsch Dipl.-Ing. K. Sparing
Dipl.-Phys.Dr. W.H. Röhl Patentanwälte
Rethelstrasse 123
D-4000 Düsseldorf(DE)

(54) Gerader, blattartiger Schaft für eine Gelenkendoprothese.

(57) Um ein Lostrennen des implantierten Prothesenschaftes (1) von seiner Umgebung bei einer gegebenenfalls notwendigen Reoperation zu erleichtern, sind die Breitseiten (9) des Schaftes (1) mit Längsnuten (10) versehen, die über die ganze Höhe des Schaftblattes verlaufen und insbesondere am proximalen Ende des Schaftblattes durchgehend ausgebildet sind.

In diesen Längsnuten (10) kann das chirurgische Trenninstrument im allgemeinen ein Meissel während des Einschlagens geführt werden, wodurch die Gefahr von Perforationen des Femurs, beispielsweise infolge eines Abrutschens des Meissels, verringert wird.

Fig.1

P. 5670/Wg/IS

Gebrüder Sulzer, Aktiengesellschaft, Winterthur/Schweiz
Protek AG, Stadtbachstr. 64, Bern/Schweiz

---

Gerader, blattartiger Schaft für eine Gelenkendoprothese

---

Die Erfindung betrifft einen geraden, blattartigen Schaft
für eine Gelenkendoprothese, insbesondere eine Hüftgelenkprothese, dessen Blatt sich vom distalen freien Ende
symmetrisch zu der Längsmittelachse konisch erweitert und
in seinen Breitseiten in Richtung der Längsmittelachse
verlaufende Längsnuten hat.

Prothesenschäfte der genannten Art für die Verankerung von
Knochenimplantaten, insbesondere von Hüftgelenksprothesen,
sind beispielsweise bekannt aus der CH-PS 622 423. Schäfte
dieser Art dienen vor allem zur zementfreien oder zumindest
zementarmen Verankerung einer Prothese, wobei bei einer
zementarmen Verankerung der als Füllmaterial dienende
Knochenzement von einer tragenden Funktion entlastet ist
und auch bei dieser Verankerungsart die tragende Abstützung
weitestgehend durch Verklemmen des Schaftes im Knochen
erfolgt.

Die Implantation derartiger Schäfte muss dabei bekanntlich
so erfolgen, dass die Breitseiten der Schaftblätter senkrecht zur Achse des Kniegelenkes verlaufen. Aus dieser
Forderung folgt, dass im allgemeinen der blattartige Schaft
exzentrisch im Femurknochen sitzt. Weiterhin ist ganz allgemein bei Implantaten die Möglichkeit einer späteren Reoperation nicht vollständig auszuschliessen. Bei einer
derartigen Reoperation erfolgt die Trennung des Implantatschaftes von dem an- oder - besonders wenn der Schaft mit Durchbrüchen versehen ist - eingewachsenen Gewebe mit Hilfe eines scharfen meisselartigen Instruments. Infolge der unsymmetrischen Lage des Implantates im Femur besteht eine erhöhte Gefahr, dass bei dieser Trennung Perforationen des Femurknochens, beispielsweise bei einem Abrutschen des Meissels beim Einschlagen, hervorgerufen werden.

Aufgabe der Erfindung ist es, diese Gefahr zu verringern und einen Prothesenschaft zu schaffen, an dem das Trenninstrument während des Lostrennens des Implantats vom Knochengewebe - bzw. evtl. vom Zementbett - geführt werden kann. Mit der vorliegenden Erfindung wird diese Aufgabe dadurch gelöst, dass die Längsnuten bis zum proximalen ·Ende des Schaftes durchgehen.

Bei dem neuen Schaft kann der Operateur für die Trennung des Schaftes von seiner Umgebung den Trennmeissel am proximalen Ende des Schaftes in den Längsnuten ansetzen und - da der Schaft ja voraussetzungsgemäss darüberhinaus gerade ist - senkrecht nach unten einschlagen, wobei der Meissel in den Längsnuten geführt wird; deren Form und Abmessungen sind daher mit Vorteil an diejenigen des Meissels angepasst. Weiterhin wird die Führung des Meissels erleichtert, wenn die Längsnuten parallel zur Längsmittelachse verlaufen.

Eine erhöhte Bedeutung erhält die erfindungsgemässe Ausbildung des Schaftes, wenn mindestens die Oberflächen der Breitseiten aus einem bioaktiven Material bestehen und/oder der Schaft mit in den Längsnuten angeordneten Durchbrüchen versehen ist.

Um am proximalen Ende des Schaftes ein Einwachsen des Gewebes von oben in die durchgehenden Längsnuten zu erschweren, ist es weiterhin zweckmässig, wenn die laterale
Schmalseite des Schaftes im oberen proximalen Bereich
der Schafthöhe parallel zur Längsmittelachse verläuft;
diese Massnahme hat den zusätzlichen Vorteil, dass die
Uebertragung der Rotationskräfte vom Trochanter auf das
Implantat und umgekehrt verbessert wird.

Im folgenden wird die Erfindung anhand eines Ausführungsbeispiels im Zusammenhang mit der Zeichnung näher erläutert.

Fig. 1 ist ein Seitenansicht auf eine Breitseite eines
Schaftes für eine Hüftgelenkprothese;

Fig. 2 ist der Schnitt II-II von Fig. 1;

Fig. 3 stellt eine Aufsicht auf Fig. 1 von oben dar,
während die

Fig. 4 - 6 die Schnitte IV-IV bis VI-VI von Fig. 1
wiedergeben.

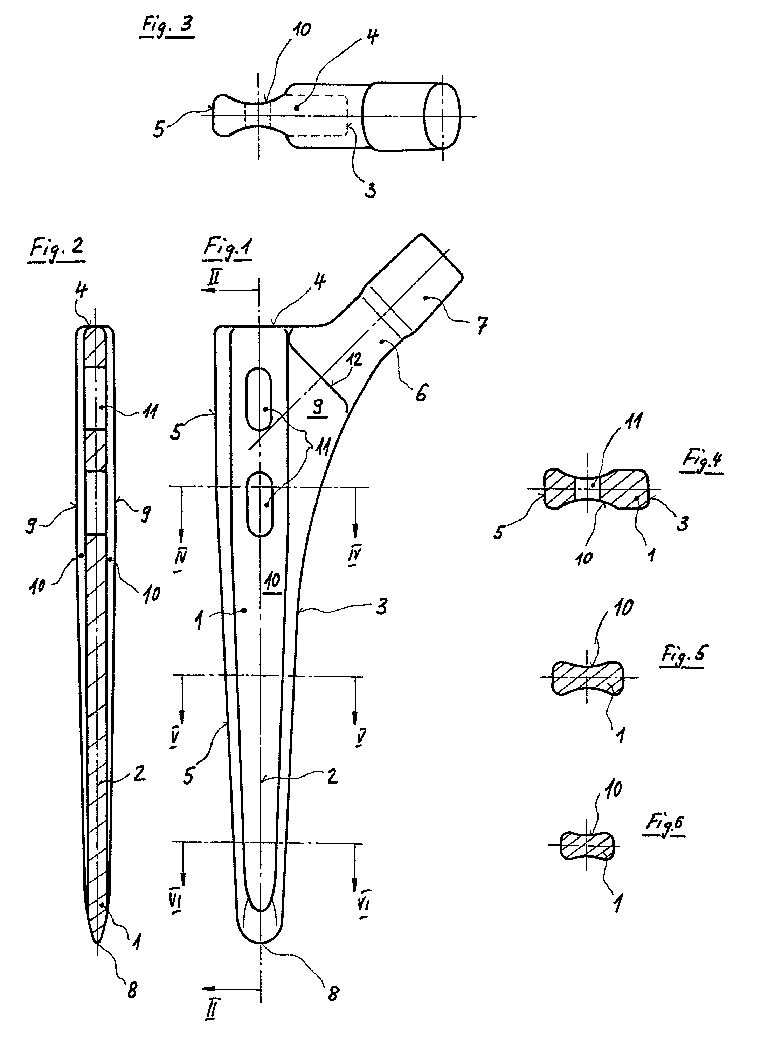

Der Prothesenschaft 1 (Fig. 1) erweitert sich von seinem
distalen Ende 8 nach allen Seiten konisch, wobei der Konus
symmetrisch zu einer Längsmittelachse 2 ausgebildet ist.
Die mediale Schmalseite 3 des Konus geht in einen Bogen
über, der in einem Hals 6 endet; auf diesen ist ein sich
nach aussen konisch verjüngender Zapfen 7 aufgesetzt, der
den nicht gezeigten kugelförmigen Gelenkkopf aufnimmt. Die
Achse des Zapfens 7 schneidet die Längsmittelachse des
Schaftes unter einem Winkel, der im wesentlichen dem Winkel
zwischen dem Schenkelhals und der Femurachse eines natürlichen Hüftgelenkes entspricht und im vorliegenden Fall
45$^\circ$ beträgt.

Bei der lateralen Schmalseite 5 des Schaftes 1 reicht die
konische Erweiterung bis auf etwa 2/3 bis 3/4 der Schafthöhe; von dort aus verläuft die laterale Schmalseite 5 parallel zur Längsmittelachse 2 und endet am proximalen Ende des Schaftes 1 in einer horizontalen Oberfläche 4. Von der Oberfläche 4 ausgehend sind erfindungsgemäss in den beiden Breitseiten 9 des Schaftes 1 als Hohlkehlen ausgebildete Längsnuten 10 vorgesehen, die die ganze Schafthöhe parallel zur Längsmittelachse 2 durchsetzen im Gegensatz zu bekannten Konstruktionen, bei denen die Längsnuten parallel zur Oberfläche der Breitseiten konisch verlaufend ausgebildet sind. In den Längsnuten 10 sind Durchbrüche 11 vorgesehen, durch die Knochengewebe hindurchwachsen kann.

An den Breitseiten 9 ist zwischen dem Prothesenhals 6 und dem Schaftblatt ein Absatz 12 vorgesehen.

Als Materialien für den neuen Prothesenschaft eigenen sich alle in der Implantat-Technik üblichen metallischen oder keramischen Werkstoffe sowie die für Knochenimplantate bekannten Kunststoffe; dabei können die metallischen Werkstoffe zusätzlich mit gegebenenfalls bioaktiven Beschichtungen - wie z.B. Metalloxiden oder -nitriden, insbesondere Titanoxid bzw. -nitrid - versehen sein.

Patentansprüche

1. Gerader, blattartiger Schaft für eine Gelenkendoprothese, insbesondere eine Hüftgelenkprothese, dessen Blatt sich vom distalen freien Ende symmetrisch zu der Längsmittelachse konisch erweitert und in seinen Breitseiten in Richtung der Längsmittelachse verlaufende Längsnuten hat, dadurch gekennzeichnet, dass die Längsnuten (10) bis zum proximalen Ende des Schaftes (1) durchgehen.

2. Schaft nach Anspruch 1, dadurch gekennzeichnet, dass die Längsnuten (10) parallel zur Längsmittelachse (2) verlaufen.

3. Schaft nach Anspruch 1, dadurch gekennzeichnet, dass die laterale Schmalseite (5) des Schaftes (1) im oberen proximalen Bereich der Schafthöhe parallel zur Längsmittelachse (2) verläuft.

4. Schaft nach Anspruch 1, dadurch gekennzeichnet, dass mindestens die Oberflächen der Breitseiten (9) aus einem bioaktiven Material bestehen und/oder der Schaft (1) mit in den Längsnuten angeordneten Durchbrüchen (11) versehen ist.

5. Schaft nach Anspruch 1, dadurch gekennzeichnet, dass Form und Abmessungen der Längsnuten (10) mindestens annähernd an diejenigen eines chirurgischen Meissels angepasst sind.

Fig. 3

Fig. 2

Fig. 1

Fig. 4

Fig. 5

Fig. 6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl ³) |
|---|---|---|---|
| Y | --- <br> US-A-3 815 599 (BEYERLE) <br> * Figur 1; Spalte 4, Zeilen 8-34; Spalte 5, Zeilen 61-68; Spalte 6, Zeilen 1-25 * | 1-4 | A 61 F 1/03 |
| Y,D | --- <br> GB-A-2 005 546 (MIEDERER) <br> * Figuren 1,2,4 * | 1-4 | |
| A | --- <br> DE-A-2 627 569 (GRISS et al.) <br><br> * Figuren 1,2,5-11; Seite 7, Zeilen 12-14 * <br><br> ----- | | |

| | | RECHERCHIERTE SACHGEBIETE (Int. Cl. ³) |
|---|---|---|
| | | A 61 F |

Der vorliegende Recherchenbericht wurde für alle Patentansprüche erstellt.

| Recherchenort <br> DEN HAAG | Abschlußdatum der Recherche <br> 11-02-1983 | Prüfer <br> FISCHER G.H. |
|---|---|---|

EPA Form 1503 03.82

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument